# EUROPÄISCHE PATENTSCHRIFT

(11) **EP 3 461 528 B1**
(45) Veröffentlichungstag und Bekanntmachung des Hinweises auf die Patenterteilung: **07.04.2021**
(21) Anmeldenummer: 18193774.9
(22) Anmeldetag: 11.09.2018
(51) Int. Cl.: A61M 39/28, A61M 5/142, A61M 5/168

(54) **SCHLAUCHKLEMME UND VOLUMETRISCHE PUMPE MIT SCHLAUCHKLEMME**
HOSE CLAMP AND VOLUMETRIC PUMP WITH HOSE CLAMP
COLLIER DE SERRAGE ET POMPE VOLUMÉTRIQUE POURVU DE COLLIER DE SERRAGE

(30) Priorität: 28.09.2017 DE 102017122647
(43) Veröffentlichungstag der Anmeldung: 03.04.2019
(73) Patentinhaber: B. Braun Melsungen AG, 34212 Melsungen (DE)
(72) Erfinder: KRAMER, Matthias, 34212 Melsungen (DE); JACOBSKÖTTER, Sarah, 24105 Kiel (DE); STEGER, Jürgen, 34327 Körle (DE); FREIGANG, Helmut, 34327 Körle (DE); SCHRÖDL, Christian, 70182 Stuttgart (DE); BATZDORF, Stefan, 36041 Fulda (DE)
(74) Vertreter: Winter, Brandl - Partnerschaft mbB

(56) Entgegenhaltungen:
- EP-B1- 2 583 716
- WO-A1-2006/081866
- DE-A1-102013 014 217
- DE-A1-102014 018 164
- DE-A1-102015 117 493
- GB-A- 2 439 325

## Beschreibung

### Technischer Hintergrund

Die vorliegende Erfindung betrifft eine medizinische Schlauchklemme, insbesondere eine Infusionsschlauchklemme, zum (dichtenden) Klemmen bzw. Abklemmen eines flexiblen medizinischen Schlauchs, insbesondere eines Infusionsschlauchs für insbesondere den Einsatz in einer Infusionspumpe, mit einem Schlauchaufnahmebereich, der dafür angepasst ist, den medizinischen Schlauch aufzunehmen, einem ersten Klemmabschnitt (Oberschale) mit einer ersten Klemmbacke, der gegenüber einem zweiten Klemmabschnitt (Unterschale) mit einer zweiten Klemmbacke bewegbar ist und einem Verschlusssystem zum sicheren Positionieren der beiden Klemmabschnitte in mehr als einer Rast-Stellung zueinander, so dass in einer (unverrasteten) Einlegeposition der beiden Klemmabschnitte der medizinischen Schlauch bzw. ein Schlauchabschnitt hiervon (ohne jegliche notwendige weitere Änderungen an einem Schlauchende, beispielsweise ohne ein vorheriges Abstöpseln von einem Wirkmittelreservoir um den Schlauch durch eine Öffnung hindurchzuschieben) in die Schlauchklemme in deren Schlauchaufnahmebereich einlegbar und entnehmbar ist, in einer (verrasteten) Offen-Raststellung der eingelegte medizinische Schlauch nicht entnehmbar ist und nicht geklemmt wird und in einer (verrasteten) Klemm-Raststellung der medizinische Schlauch zwischen der ersten Klemmbacke und der zweiten Klemmbacke (dichtend) (ab-) geklemmt/gequetsch wird. Daneben betrifft die Erfindung eine volumetrische Pumpe gemäß dem Oberbegriff des nebengeordneten Anspruchs.

### Stand der Technik

Aus dem Stand der Technik sind diverse Schlauchklemmen und Infusionsschlauchpumpen bekannt. Meist handelt es sich hierbei um Verdrängungspumpen die von außen her ein Volumen in einem flexiblen Schlauch (Einmalartikel) verdrängen und somit eine Kontamination der Pumpenmimik mit dem in dem Schlauch geführten Medium vermeiden. Auf dem Gebiet der Infusionstechnik ist es von immanenter Bedeutung, dass ein Schlauch, der eine Verbindung zwischen einem Patienten und einem Wirkstoffreservoir herstellt, niemals geöffnet sein darf, ohne dass die Durchflussrate kontrolliert wird, da dem Patienten ansonsten in einer sogenannten "Free-Flow-Situation" eine unkontrollierte Menge an Wirkstoff zugeführt werden könnte. Bei oben genannten Pumpen besteht deshalb die Notwendigkeit, den Fluss durch den Einmalartikel zu unterbrechen, wenn die Pumpe, bspw. zum Austausch des Einmalartikels, geöffnet wird. Hierzu wird üblicherweise dem Anwender vorgeschrieben (bspw. über eine Signalausgabe an einem Display der Infusionspumpe) vor dem Öffnen der Pumpe eine Sicherheitsklemme/Schlauchklemme/Infusionsschlauchklemme, bspw. eine Rollenklemme, manuell zu schließen. In dem Fall, dass der Anwender das Schließen der Sicherheitsklemme vergisst oder die Sicherheitsklemme nicht korrekt schließt, entsteht eine Free-Flow-Situation, die den Patienten gefährdet.

Aus der EP 2 583 716 B1 ist beispielsweise eine gattungsgemäße Infusionsschlauchklemme/Schlauchklemme/Klemme zum Einsetzen in eine Infusionspumpe bekannt. Diese Klemme weist einen Grundkörper mit einem Infusionspumpenaufnahmebereich sowie einen ersten und einen zweiten Klemmschenkel zum Quetschen eines Infusionsschlauchs auf, wobei die beiden Klemmschenkel an einem ersten Infusionsschlauchklemmende schwenkbar miteinander verbunden sind. Über eine Schnappeinrichtung/Schnappverschlusssystem können die beiden Klemmschenkel in mehr als einer Lage zueinander sicher positioniert werden.

Die DE 10 2013 014 217 A1 offenbart eine medizinische Schlauchklemme mit einem zweistufigen Rastmechanismus sowie einem Lösegetriebe, welches bei einer manuellen Betätigung, ohne den Rastmechanismus zu entrasten den Verschluss des Schlauches aufhebt, solange die manuelle Betätigung anhält.

Die GB 2 439 325 A offenbart eine Klemme, die zwischen einer vollständig geöffneten Position, einer Klemmposition und einer Zwischenposition zwischen der Klemmposition und der vollständig geöffneten Position bewegt werden kann. Zwei Arme sind elastisch miteinander verbunden, wobei ein Arm durch eine Öffnung in dem Arm steht bzw. geht. Die Klemme kann in der Klemmposition (durch den Eingriff zwischen einer Kante der Öffnung und der Verzahnung) und in der Zwischenposition verrastet werden. Die Klemme ist von der Klemmposition in die Zwischenposition lösbar, ohne dass der eine Arm aus dem anderen Arm herausgezogen wird. Ein Arm weist dabei eine elastische Druckschwinge auf, welche einen eingelegten Schlauch abklemmt.

Die DE 10 2015 1177 493 A1 offenbart eine medizinische Schlauchklemme, die einen Sensor in einer Aufnahme umfasst, um zu erfassen, ob sich Klemmabschnitte in einer geschlossenen Relativposition befinden

Einschlägige Technik ist auch in der EP 2 583 716 B1 offenbart, das eine medizinische Schlauchklemme vorschlägt, mit Quetschkante, Quetschkantenauflage, Schlauchführungsraum und Verschlusssystem.

Ein Problem bei dem Stand der Technik tritt bei einem Schließen der Klemme zu Tage. Aufgrund der immer weiter ansteigenden Druckkraft mit zunehmendem Schwenken bzw. Schließen der beiden Klemmschenkel gegeneinander um den Schlauch zu Quetschen, entstehen oftmals hohe Kraft- bzw. Spannungsspitzen. Diese Kausalität rührt unter anderem auch daher, dass die medizinischen Schläuche nicht immer exakt gleich ausgebildet sind oder unterschiedliche Schläuche mit unterschiedlichen geometrischen Abmessungen und Elastizitätsmodulen in der Schlauchklemme verwendbar sind. Auch ist eine Fertigung der Schlauchklemme mit immer exakt gleichen Toleranzen schwierig und kostenaufwendig. Zum einen erfordert diese Kraftspitze einen hohen Kraftaufwand eines Bedienpersonals zum Schließen der Schlauchklemme und stellt des Weiteren ein großes Sicherheitsrisiko dar, da das Schnappverschlusssystem in einem ungünstigen Fall nicht komplett verrastet und sich ungewollt wieder lösen kann, so dass ungewolt eine gefährliche "Free-Flow-Situation" entstehen kann. Hinzu kommt, dass es Infusionspumpen mit Schlauchklemmen gibt, welche über eine schwenkbare Tür die Schlauchklemme automatisch schließen, was eine optische oder akustische Kontrolle einer sicheren Verrastung des Verschlusssystems weiter erschwert.

### Zusammenfassung der Erfindung

Es ist daher die Aufgabe der Erfindung, die Nachteile aus dem Stand der Technik zu vermeiden oder wenigstens zu mindern und Mittel und Wege bereitzustellen, die eine Erhöhung der Betriebssicherheit einer Schlauchklemme als auch einer volumetrischen Pumpe ermöglichen. Ein bevorzugtes Ziel der vorliegenden Erfindung ist es, das Einlegen und das Klemmen eines flexiblen medizinischen Schlauchs in einer Schlauchklemme als auch in einer volumetrische Pumpe sicherer und einfacherer zu gestalten.

Diese Aufgaben und Ziele werden hinsichtlich einer gattungsgemäßen Schlauchklemme erfindungsgemäß dadurch gelöst, dass die erste Klemmbacke über ein Verbindungsglied elastisch/federnd beweglich zu dem ersten Klemmabschnitt und/oder die zweite Klemmbacke über ein Verbindungsglied elastisch/federnd beweglich zu dem zweiten Klemmabschnitt ausgebildet ist.

Grundsätzlich sieht die vorliegende Erfindung eine (teilweise) Entkopplung bzw. eine elastisch/federnd bewegliche Kopplung zwischen dem Klemmabschnitt und der Klemmbacke über das Verbindungsglied vor. Durch eine konstruktive Ausgestaltung der Schlauchklemme lässt sich das zumindest eine Verbindungsglied realisieren, welches bei einem Kraftfluss in Serie zwischen der ersten Klemmbacke und der zweiten Klemmbacke geschaltet ist. So lässt sich in Gesamtbetrachtung des Kraftflusses eine Elastizität zwischen den beiden Klemmbacken einstellen, wohingegen die beiden Klemmabschnitte relativ dazu gesehen starr ausgebildet sind und in Hauptsache in Kombination mit dem Verschlusssystem der sicheren Positionierung zueinander dienen. Durch eine solche konstruktive Ausbildung der Schlauchklemme lassen sich insbesondere Kraftspitzen abfangen und die beiden Klemmabschnitte relativ zueinander sicher fixieren. Das Risiko eines ungewollten Lösens der Positionierung bzw. der Klemm-Raststellung wird weitestgehend unterbunden.

Die erfindungsgemäße Schlauchklemme erleichtert durch die (teilweise) Entkopplung bzw. elastische Kopplung von Klemmbacke und Klemmabschnitt und damit der (teilweisen) Entkopplung von Schließmechanismus (mit den beiden Klemmabschnitten und dem Verschlusssystem) und Klemmmechanismus (Klemmbacken), dass die Schlauchklemme mit einer noch größeren Unabhängigkeit von dem eingelegten medizinischen Schlauch als auch von dem Klemmmechanismus noch sicherer und einfacher komplett verrastet bzw. in die Klemm-Raststellung gebracht werden kann.

Vorteilhafte Ausführungsformen sind in den Unteransprüchen beansprucht und werden nachfolgend erläutert.

Vorzugsweise kann in der Klemm-Raststellung der erste Klemmabschnitt durch das Verschlusssystem starr/unbeweglich/unelastisch gegenüber dem zweiten Klemmabschnitt positioniert sein, insbesondere sind die beiden Klemmabschnitte durch eine geometrische Ausgestaltung mit einem entsprechenden Material relativ starr/ unelastisch (beispielsweise durch eine dicke Wandung, durch eingebrachte Streben oder Profile in Kombination mit einem geringen Elastizitätsmodul), und die erste Klemmbacke und/oder die zweite Klemmbacke sind im Wesentlichen nur in eine Klemmdruckrichtung (Richtung, in welcher die Kraft der Klemmbacke auf den eingelegten Schlauch in der Klemm-Raststellung wirkt) elastisch beweglich. Durch die elastische Beweglichkeit der Klemmbacke gegenüber dem Klemmabschnitt in lediglich der Klemmdruckrichtung (ein translatorischer Freiheitsgrad) lässt sich eine Spannungsspitze beim Klemmen abfangen, wohingegen eine ansonsten (gewollte) starre Anbindung der Klemmbacke an den Klemmabschnitt beibehalten wird.

Gemäß einem bevorzugten Aspekt können die beiden Klemmabschnitte der Schlauchklemme relativ zueinander schwenkbar ausgebildet sein. Insbesondere kann die Schlauchklemme ein Scharnier, besonders bevorzugt ein Filmscharnier, aufweisen, durch das der erste Klemmabschnitt relativ zu dem zweiten Klemmabschnitt um eine Schwenkachse schwenkbar ist, so dass die medizinische Schlauchklemme sich maulartig öffnen und schließen lässt. So kann beispielsweise das Verschlusssystem auf der dem Scharnier gegenüberliegenden Seite angeordnet werden. Vorteilhafter Weise ist die jeweilige Klemmbacke des jeweiligen Klemmabschnitts zwischen dem Verschlusssystem und der Schwenkachse bzw. dem Scharnier angeordnet.

Gemäß einem anderen Aspekt der Erfindung kann die Schlauchklemme anstelle des Scharniers auch einen Mechanismus aufweisen, mit dem der zweite Klemmabschnitt nur einen translatorischen Freiheitsgrad in Richtung des ersten Klemmabschnitts aufweist und bewegbar ist. Diese translatorische Bewegung der beiden Klemmabschnitte zu- bzw. gegeneinander ist neben der Schwenkbewegung eine weitere alternative Ausführungsform.

Gemäß einer vorteilhaften Weiterbildung kann das Verschlusssystem in Form eines Schnappverschlusssystem/Schnappeinrichtung (mit zwei Raststellungen) ausgebildet sein. Ein solches Schnappverschlusssystem erlaubt eine einfache und sichere Realisierung der zwei Rast-Stellungen Offen-Raststellung und Klemm-Raststellung der Schlauchklemme. Ein solches Schnappverschlusssystem ist aus der EP 2 583 716 B1 bekannt.

Vorzugsweise kann die erste Klemmbacke und/oder die zweite Klemmbacke als Klemmkante (mit linearer geometrischer Form) ausgebildet sein, deren Längsachse (Klemmenlängsachse) quer zu der Längsachse des eingelegten medizinischen Schlauchs verläuft. Eine solche Klemmkante/Klemmschneide/Klemmkeil ist dafür angepasst den medizinischen Schlauch optimal dichtend abzuklemmen ohne diesen zu beschädigen.

Gemäß einer bevorzugten Ausführungsform kann der erste Klemmabschnitt und/oder der zweite Klemmabschnitt einen plattenförmigen Grundkörper aufweisen, der vorzugsweise im direkt angrenzenden Bereich an die jeweilige Klemmbacke zumindest eine Aussparung, insbesondere in Form eines Langlochs, aufweist, wodurch ein Verbindungsglied im Grundkörper ausgebildet wird. Durch eine gezielte Einbringung der Aussparung kann also bereits in dem Klemmabschnitt integral das Verbindungsglied eingearbeitet werden.

Bevorzugt können die Klemmbacken als Klemmkanten ausgebildet sein und der erste Klemmabschnitt und/oder der zweite Klemmabschnitt zwei Langlöcher aufweisen, deren Längsrichtung (vom Langloch) quer zu der Klemmenlängsrichtung liegen und das Langloch jeweils vorzugsweise direkt an die jeweilige Klemmkante angrenzt. Durch diese Anordnung der Langlöcher wird die Klemmkante an zwei Seiten von dem Klemmabschnitt entkoppelt bzw. gelöst, wohingegen die verbleibenden zwei Seiten der Klemmkante mit dem Klemmabschnitt verbunden bleiben. Diese beiden verbleibenden Verbindungsbereiche/Verbindungsstellen bilden die zwei Verbindungsglieder aus, welche die Klemmbacke elastisch beweglich zum Klemmabschnitt gestalten. Insbesondere kann über deine Definition der Dicke der Verbindungsglieder die Elastizität eingestellt werden.

So ist es von Vorteil, wenn der erste Klemmabschnitt, das Verbindungsglied und die erste Klemmbacke stoffeinstückig ausgebildet sind und/oder der zweite Klemmabschnitt, das Verbindungsglied und die zweite Klemmbacke stoffeinstückig ausgebildet sind. Besonders bevorzugt ist die gesamte medizinische Schlauchklemme samt Scharnier stoffeinstückig ausgebildet. Besonders bevorzugt kann die Schlauchklemme aus Kunststoff gefertigt und weiter bevorzugt als stoffeinstückiges (Thermoplast-)Spritzgussteil ausgeführt sein, in welchem sowohl die Verbindungsglieder als auch das Scharnier integral eingearbeitet bzw. durch gezielte Materialaussparungen in einer Schauchklemmenwandfläche ausgebildet sind.

Vorzugsweise kann das Verbindungsglied ein elastisches Material, eine Feder oder ein Vorspannmechanismus sein. Bei diesen drei Elementen kann auf unterschiedliche Art und Weise eine gewünschte Elastizität definiert werden.

Ein weiterer unabhängig beanspruchter Aspekt der Erfindung betrifft eine gattungsgemäße volumetrische Pumpe, insbesondere eine Infusionspumpe, zum Fördern eines Mediums durch einen flexiblen medizinischen Schlauch, mit einem Gehäuse, wobei erfindungsgemäß an dem Gehäuse zum dichtenden (ab)klemmen des medizinischen Schlauchs eine erfindungsgemäße medizinische Schlauchklemme, vorzugsweise lösbar, befestigt ist.

### Kurzbeschreibung der Figuren

Die Erfindung wird nachstehend anhand bevorzugter Ausführungsbeispiele unter Bezugnahme der begleitenden Figuren erläutert. Die Figuren sind lediglich schematischer Natur und dienen ausschließlich dem Verständnis der Erfindung. Gleiche Elemente sind mit denselben Bezugszeichen versehen. Die Merkmale der verschiedenen Ausführungsbeispiele können untereinander ausgetauscht werden. Es zeigen:
- Fig. 1: eine perspektivische Darstellung einer erfindungsgemäßen Schlauchklemme einer erste bevorzugte Ausführungsform mit eingelegtem medizinischen Schlauch, wobei die Schlauchklemme dabei in eine erfindungsgemäße volumetrische Pumpe einer ersten Ausführungsform eingesetzt ist;
- Fig. 2: eine perspektivische Darstellung der Schlauchklemme der ersten bevorzugten Ausführungsform in einer unverrasteten Einlegeposition, bei welcher die innere Struktur der Schlauchklemme gezeigt ist;
- Fig. 3: eine perspektivische Darstellung der Schlauchklemme der ersten bevorzugten Ausführungsform in einer unverrasteten Einlegeposition, bei welcher die äußere Gestaltung der Schlauchklemme gezeigt ist;
- Fig. 4: eine perspektivische Darstellung der Schlauchklemme der ersten bevorzugten Ausführungsform in einer Offen-Raststellung;
- Fig. 5: eine perspektivische Darstellung der Schlauchklemme der ersten bevorzugten Ausführungsform in einer Klemm-Raststellung;
- Fig. 6: eine Längsschnittdarstellung der Schlauchklemme aus Fig. 5; und
- Fig. 7: eine Längsschnittdarstellung einer erfindungsgemäßen Schlauchklemme einer zweiten bevorzugten Ausführungsform.

### Detaillierte Beschreibung bevorzugter Ausführungsformen

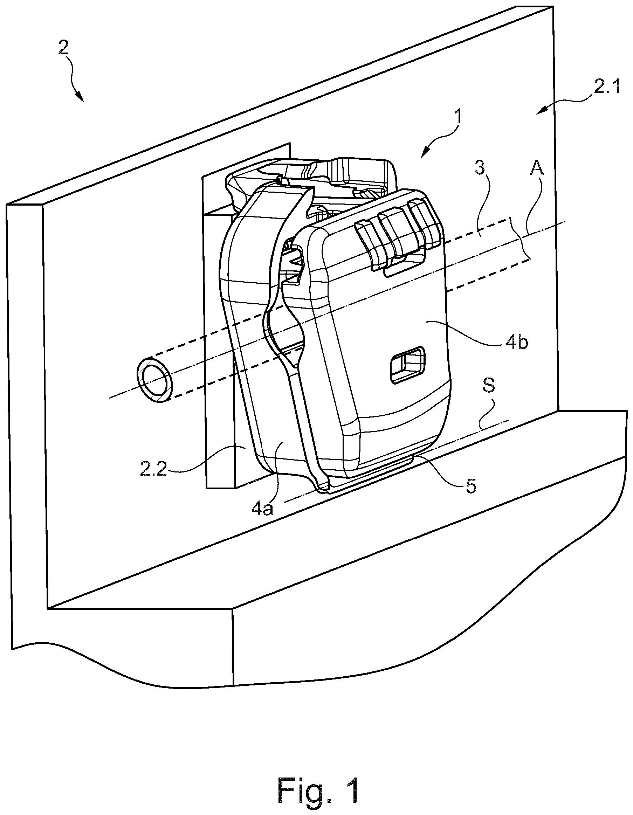

Figur 1 zeigt schematisch eine perspektivische Darstellung einer erfindungsgemäßen medizinischen Schlauchklemme 1, genauer gesagt einer Infusionsschlauchklemme 1 einer ersten bevorzugten Ausführungsform, welche in eine erfindungsgemäße volumetrische Pumpe 2 in Form einer Infusionspumpe 2 (hier schematische Teildarstellung) mit einem zugehörigen Gehäuse 2.1 eingesetzt ist. Ein flexibler medizinischer Schlauch 3 (hier schematische Teildarstellung), der als Infusionsschlauch 3 ausgebildet ist, ist in der Infusionsschlauchklemme 1 klemmend verrastet aufgenommen (Klemm-Raststellung). Bei der schematisch dargestellten Infusionspumpe 2 handelt es sich um eine Pumpe mit Schieberperistaltik, bei welcher der Infusionsschlauch 3, der einen Patienten mit einem Wirkmittelreservoir (nicht dargestellt) verbindet, vor den in einer mit einer Pumpenklappe (nicht dargestellt) verschließbaren Schlaucheinlegöffnung angeordneten Peristaltikschiebern (nicht dargestellt) platziert wird. Die Pumpenklappe wird anschließend geschlossen und dient als Gegenlager zu den Peristaltikschiebern, welche in einer wellenförmigen Verdrängungsbewegung ein Volumen im Inneren des flexiblen Infusionsschlauchs 3 in eine definierte Förderrichtung schieben.

Bei solchen medizinischen Schlauchklemmen 1 als auch bei solchen Infusionspumpen 2 muss der als Einmalartikel ausgeführte flexible Infusionsschlauch 3 häufig neu eingelegt oder gewechselt werden. Ein solcher Einlege-/Wechselvorgang ist sehr aufwendig, da der Infusionsschlauch 3 exakt vor der Pumpenmimik (nicht dargestellt) positioniert werden muss, damit überhaupt eine Volumenförderung stattfinden kann. Ziel der vorliegenden Erfindung ist unter anderem, den Einlege-/Wechselvorgang des Infusionsschlauch 3 für das Bedienpersonal einfacher und sicherer zu gestalten, ein korrektes Einlegen und sicheres Verrasten bzw. Klemmen des Infusionsschlauchs 3 durch konstruktive Maßnahmen zu unterstützen und vorzugsweise ein einhändiges und schonendes Einlegen/Wechseln des Schlauchs zu ermöglichen. Dieses Ziel wird durch die erfindungsgemäße Schlauchklemme 1 erreicht, welche im Folgenden näher erläutert wird.

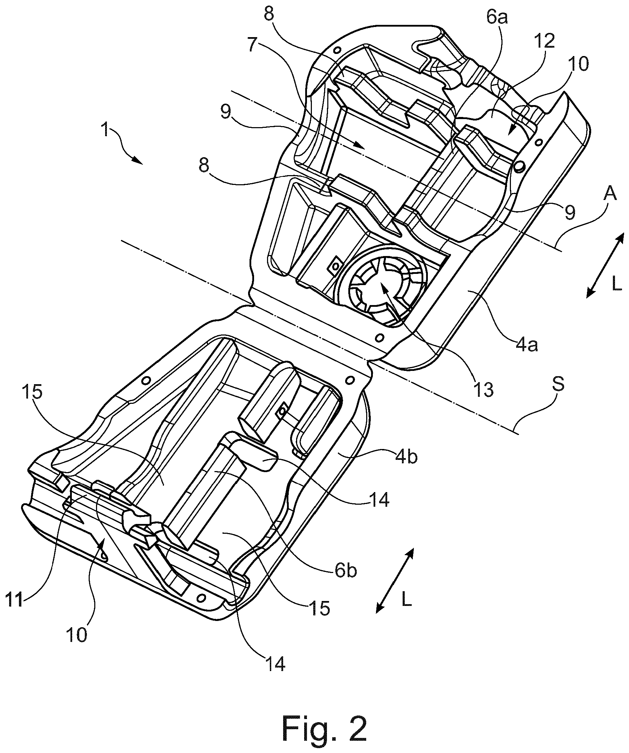

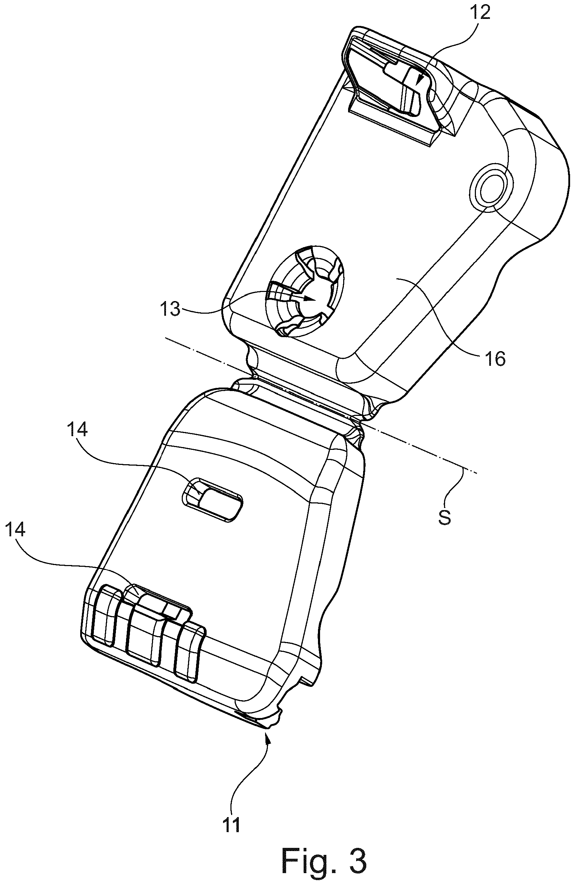

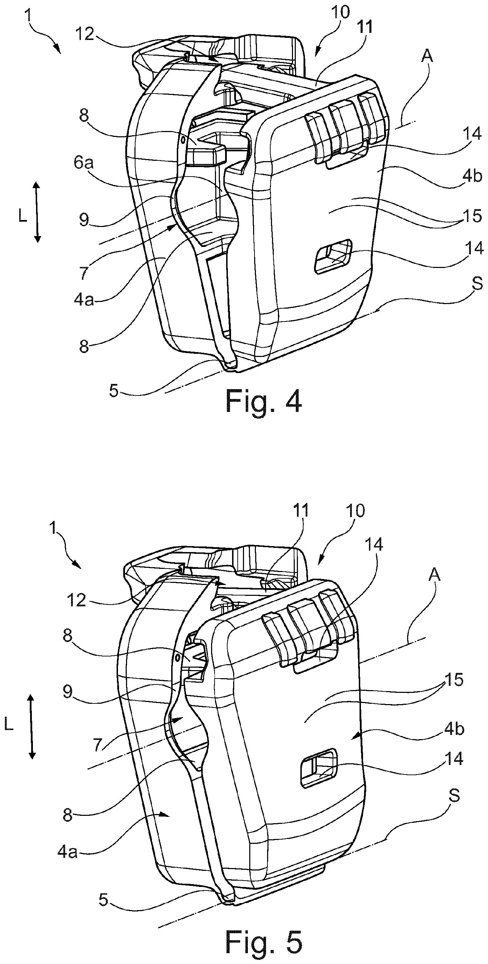

Die Figuren 2 bis 6 zeigen die Schlauchklemme 1 der ersten bevorzugten Ausführungsform. In den Figuren 2 und 3 wird die Schlauchklemme 1, welche sich in einer unverrasteten Einlegeposition befindet, genauer dargestellt. Die Schlauchklemme 1 weist einen ersten Klemmabschnitt 4a und einen zweiten Klemmabschnitt 4b auf, welche gegeneinander geschwenkt werden können. Hierfür sind die beiden Klemmabschnitte 4a, 4b an einem Ende durch ein (Film-) Scharnier 5 um eine Schwenkachse S schwenkbar miteinander verbunden. Beide Klemmabschnitte 4a, 4b können so vorteilhafter Weise bereits mit dem Scharnier 5 verbunden als ein einziges/stoffeinstückiges Spritzgussteil aus Kunststoff gefertigt werden. Der erste Klemmabschnitt 4a und der zweite Klemmabschnitt 4b weisen einen schalenförmigen Grundkörper mit jeweils einer Trapezkontur auf, wobei die Trapezkontur bzw. der Außenumriss des ersten Klemmabschnitts 4a symmetrisch zu dem zweiten Klemmabschnitt 4b ausgebildet ist, so dass die beiden Klemmabschnitte 4a, 4b in einer Klemm-Raststellung (siehe Figur 1 und 5), ähnlich eines Koffers oder einer Muschel, einen einheitlichen (in Draufsicht trapezförmigen) Korpus bilden.

Der erste Klemmabschnitt 4a weist in einem mittig gelegenen Bereich des trapezförmigen Korpus (zwischen dem Scharnier 5 und der gegenüberliegenden Seite angeordnet) eine erste Klemmbacke 6a auf, die in Form einer Klemmkante/Klemmschneide ausgebildet ist. Die Klemmkante 6a steht dabei senkrecht von dem ersten Klemmabschnitts 4a, der den ersten schalenförmigen Grundkörper der Schlauchklemme 1 bildet, in Richtung des zweiten Klemmabschnitts 4b (in einer Klemm-Raststellung siehe Fig.1 und Fig. 5) hervor. Die erste Klemmkante 6a ist wandförmig ausgebildet und verläuft linear in eine Klemmenlängsrichtung L, welche quer zu der Schwenkachse S des Scharniers 5 liegt. Auch der zweite Klemmabschnitt 4b weist in einem mittig gelegenen Bereich seines trapezförmigen Korpus eine zweite Klemmbacke 6b in Form einer Klemmkante auf. Diese zweite Klemmkante 6b ist keilartig ausgebildet und verläuft in der Klemm-Raststellung (siehe Fig. 1 und Fig. 5) ebenfalls linear in die Klemmenlängsrichtung L und damit auch quer zu der Schwenkachse S. Die erste Klemmkante 6a und die zweite Klemmkante 6b sind mit gleichem Abstand zur Schwenkachse S sowie von ihrer Ausdehnung her in Klemmenlängsrichtung L gesehen symmetrisch ausgebildet. Da die beiden Klemmkanten 6a, 6b auch auf gleicher Höhe der Schwenkachse S liegen, bewegen sich bzw. schwenken die beiden Klemmkanten 6a, 6b bei einem Schwenken/Schließen der Klemmabschnitte 4a, 4b um die Schwenkachse S aufeinander zu, um einen Infusionsschlauch 3 zwischen sich zu klemmen.

Die Schlauchklemme 1 weist einen Schlauchaufnahmebereich 7 für eine Aufnahme des Infusionsschlauchs 3 auf. Genauer gesagt weist in dieser Ausführungsform der ersten Klemmabschnitt 4a den Schlauchaufnahmebereich 7 auf. Hierfür ist in Klemmenlängsrichtung L gesehen an den Enden der ersten Klemmkante 6a jeweils ein Steg 8 mit Vorsprüngen ausgebildet, der parallel zur Schwenkachse S bzw. senkrecht zur Klemmenlängsrichtung L verläuft und zum zweiten Klemmabschnitt in der Klemm-Raststellung vom ersten Klemmabschnitt 4a her absteht. Zwischen diesen zwei Stegen 8 kann der Infusionsschlauch 3 eingelegt werden. Die erste Klemmkante 6a (Klemmenlängsachse L) steht dabei quer auf einer Schlauchlängsachse A des eingelegten Infusionsschlauchs 3. Zudem sind an den Durchtritten des zwischen den zwei Stegen 8 eingelegten Infusionsschlauchs 3 durch die Seitenflächen des ersten Klemmabschnitts 4a teilkreisförmige Ausnehmungen/Öffnungen/Aussparungen/Kuhlen 9 vorgesehen, so dass sich der runde Infusionsschlauch 3 in diese einpasst.

An den in Klemmenlängsrichtung L gesehen oberen bzw. dem Scharnier 5 abgewandten Enden der Klemmabschnitte 4a, 4b ist ein Verschlusssystem 10 in Form eines Schnappverschlusssystems angeordnet, mittels welchem die Schlauchklemme 1 die zwei Rast-Stellungen, nämlich die Offen-Raststellung und die Klemm-Raststellung realisiert. Genauer gesagt weist das Schnappverschlusssystem 10 einen federnden Schnapphaken 11 auf, welcher, ausgehend von einer in Fig. 2 gezeigten unverrasteten Einlegeposition, bei einem Schwenken der beiden Klemmabschnitte 4a, 4b zueinander in eine Schließrichtung über eine Anlaufschräge unter Überwindung seiner materialeigenen Elastizität quer zu seiner Längsrichtung ausgelenkt wird, um hiernach in eine Schnappöse 12 einzugreifen. Ebenso kann der Schnapphaken 11 unter Überwindung seiner materialelastischen Elastizität aus diesem Eingriff gelöst werden, um die Schlauchklemme 1 wieder in die unverrasteten Einlegeposition zu bringen bzw. zu bewegen. Das Schnappverschlusssystem 10 wird nachfolgend unter Zuhilfenahme der Figuren 4 und 5 näher beschrieben.

Der erste Klemmabschnitt 4a weist darüber hinaus ein Druckknopfloch 13 angrenzend zum Filmscharnier 5 bzw. nahe zur Schwenkachse S auf, um die Schlauchklemme 1 an der Infusionspumpe 2 (siehe Fig. 1) lösbar zu befestigen. Die Anordnung des Druckknopflochs 13 nahe der Schwenkachse S soll ein versehentliches Lösen de Schlauchklemme 1 bei einem Öffnen der Schlauchklemme 1 unterbinden bzw. zumindest minimieren. Zudem ist das Schnappverschlusssystem 10 in der Schlauchklemme 1 derart ausgebildet, dass es einen Vorsprung bildet, welcher mit einer Ausnehmung des Gehäuses 2.1 zusammenwirken kann, um eine Orientierung der um das Druckknopfloch 13 drehbaren Schlauchklemme 1 festzulegen.

Im Gegensatz zum Stand der Technik wurden in dem zweiten Klemmabschnitt 4b gezielt zwei Aussparungen 14 in Form eines länglichen Schlitzes bzw. in Form eines Langlochs eingebracht, welche parallel zu der Schwenkachse S bzw. parallel zu der Schlauchlängsachse A des Infusionsschlauchs 3 sowie quer zu der Klemmenlängsachse L verlaufen. Die zwei Langlöcher 14 sind dabei in ihren Maßen in Richtung quer zur Klemmenlängsrichtung L in etwa gleich lang und liegen sich hinsichtlich der zweiten Klemmkante 6b symmetrisch gegenüber. Genauer gesagt sind die beiden Langlöcher 14 direkt an den beiden Enden/Seiten der zweiten Klemmkante 6b angrenzend in Klemmenlängsrichtung L gesehen in den zweiten Klemmabschnitt 4b eingebracht. Konstruktiv bewirken diese beiden Langlöcher 13, dass die zweite Klemmkante 6b an zwei Seiten von dem zweiten Klemmabschnitt 4b entkoppelt ist und nur über zwei elastische Verbindungsglieder bzw. Verbindungen bzw. Verbindungsbereiche 15 mit dem zweiten Klemmabschnitt 4b verbunden ist. Diese elastischen Verbindungsglieder 15 bewirken, dass die zweite Klemmkante 6b eine definiert federnd-elastische Beweglichkeit gegenüber dem starr ausgebildeten zweiten Klemmabschnitt 4b aufweist. Genauer gesagt weist die zweite Klemmkante 6b eine konstruktiv bedingte elastische Beweglichkeit in eine Klemmdruckrichtung D (in einer Klemm-Raststellung quer zu dem zweiten Klemmabschnitt 4b und quer zu der Schlauchlängsachse A des eingelegten Infusionsschlauchs 3, siehe Fig. 1 und Fig. 5) auf. Der sich hieraus ergebende technischen Vorteil bei einem Schwenken der beiden Klemmabschnitte 4a, 4b gegeneinander bzw. bei einem Schließen und Verrasten der Schlauchklemme 1, wird später unter Zuhilfenahme der Figuren 4 bis 6 erläutert.

Figur 3 zeigt eine weitere Ansicht der Schlauchklemme 1 aus Fig.2 in einer perspektivischen Darstellung mit Blick auf die Außenfläche. Der erste Klemmabschnitt 4a weist dabei eine plane Anlagefläche/Anlageabschnitt 16 auf, welche an dem Gehäuse 2.1 der Infusionspumpe 2 an einer komplementären planen Gegenanlagefläche 2.2 auf- bzw. anliegt (siehe Fig. 1). Die dargestellte Anlagefläche 16 dient überdies dazu, den gehäuseseitige ersten Klemmabschnitt 4a so abzustützen, dass bei einem (Ver-)Schließen der Schlauchklemme 1 und (ab-)klemmen eines in den Schlauchaufnahmebereich 7 eingelegten Infusionsschlauchs 3, lediglich der zweite Klemmabschnitt geschwenkt und in Richtung Gehäuse 2.1 (siehe Fig. 1) gedrückt werden muss, bis das Schnappverschlusssystem 10 eingreift und verrastet.

Die Funktionsweise der konstruktiv ausgebildeten elastischen Beweglichkeit der zweiten Klemmkante 6b gegenüber dem zweiten Klemmabschnitt 4b über das Verbindungsglied 15 wird nachfolgend erläutert. Figur 4 zeigt die Schlauchklemme 1 aus Fig. 1 bis Fig. 3 der ersten bevorzugten Ausführungsform in der Offen-Raststellung. Gegenüber der unverrasteten Einlegeposition aus Fig. 2 und Fig. 3, in welcher die beiden Klemmabschnitte 4a, 4b frei beweglich gegeneinander um die Schenkachse S schwenken können und in welcher der Infusionsschlauch 3 eingelegt oder auch entnommen werden kann, wurden die beiden Klemmabschnitte 4a, 4b in Schließrichtung zueinander hin geschwenkt und der Schnapphaken 11 des Schnappverschlusssystems 10 wurde über die Anlaufschräge unter Überwindung seiner materialeigenen Elastizität quer zu seiner Längsrichtung ausgelenkt und schnappte hiernach in einer Zwischenposition (Offen-Raststellung) in die Schnappöse 12 ein (verrastet über einen Hinterschnitt). In dieser Offen-Raststellung ist der eingelegte Infusionsschlauch 3 (hier der Übersichthalber nicht dargestellt, siehe dafür Fig. 1) in dem Schlauchaufnahmebereich so eingelegt, dass dieser nicht ohne Öffnung des Schnappverschlusssystems 10 wieder entnommen werden kann, wohingegen der Infusionsschlauch 3 aber nicht geklemmt wird (Zustand eines freigegebenen Flusses des Fluides durch den Infusionsschlauch 3).

Wird nun der zweite Klemmabschnitt 4b weiter in Schließrichtung gegenüber dem ersten Klemmabschnitt 4a geschwenkt, so pressen/quetschen/klemmen die beiden sich gegenüberliegenden Klemmbacken 6a, 6b den zwischen ihnen angeordneten flexiblen und elastischen Infusionsschlauch 3 immer weiter ab. Ab einem speziell definierten Schwenkwinkel, welcher auch abhängig von der Geometrie und dem Material des Infusionsschlauchs 3 ist, wird ein Fluss durch den Infusionsschlauch 3 komplett abgeklemmt. Der Schnapphaken 11 des Schnappverschlusssystems 10 rastet nach Überwindung einer weiteren Anlauffläche und unter Überwindung seiner Eigenelastizität ab einem bestimmten Punkt über einen Hinterschnitt am ersten Klemmabschnitt 4a ein und klemmt so in der Klemm-Raststellung den Infusionsschlauch 3 sicher ab. Aufgrund der beiden eingebrachten Langlöcher 14 und der zwei ausgebildeten Verbindungsglieder 15 zu beiden Seiten der zweiten Klemmkante 6b wird bei einem Schließen eine Kraftspitze abgebaut und ein sicheres Verrasten des Schnappverschlusssystems gewährleistet, da die zweite Klemmkante 6b elastisch beweglich zu dem zweiten Klemmabschnitt 4b ist. Während in direkter Auflage der zweiten Klemmkante 6b auf den bereits (komplett) zusammengequetschen Infusionsschlauch 3 eine weitere Relativbewegung in die Klemmdruckrichtung D nur sehr schwer möglich ist, kann hingegen der zweite Klemmabschnitt 4b, aufgrund der (teilweisen) Entkopplung bzw. seriellen Schaltung/Kopplung über die elastischen Verbindungsglieder 15, weiter gegen den ersten Klemmabschnitt 4a gedrückt und über das Schnappverschlusssystem in der Klemm-Raststellung sicher verrastet werden.

Figur 5 zeigt diese Klemm-Raststellung der Schlauchklemme 1, bei welcher der Schnapphaken 11 des Schnappverschlusssystems 10 verrastet ist und der Infusionsschlauch 3 (siehe Fig. 1) abgequetscht ist.

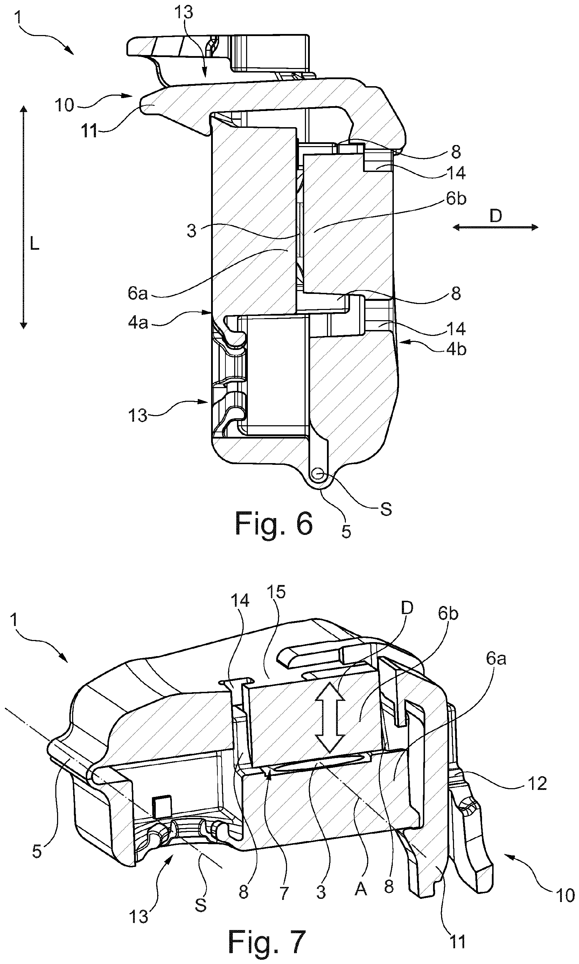

Figur 6 zeigt in einem Längsschnitt durch die Klemmkanten 6a und 6b detailliert die Funktionsweise der Verbindungsglieder 15. Während die Klemmkanten 6a und 6b den Infusionsschlauch 3 zwischen sich quetschen und abklemmen, ist der Schnapphaken 11 über den Hinterschnitt an dem zweiten Klemmabschnitt 4b verrastet. Die beiden Klemmabschnitte 4a, 4b sind im Vergleich gegenüber den Klemmkanten 6a, 6b starr gegeneinander fixiert. Ein Kraftfluss verläuft ausgehend von der ersten Klemmkante 6a über den starr an der ersten Klemmkante 6a angebundenen ersten Klemmabschnitt 4a über zwei Wege, nämlich über das Schnappverschlusssystem 10 (insbesondere Schnapphaken 11) und über das Scharnier 5 auf den zweiten Klemmabschnitt 4b über und vor hier aus (seriell), unter Zwischenschaltung der elastische Verbindungsglieder 15, auf die zweite Klemmkante 6b. Erfindungsgemäß sind also im Gegensatz zum Stand der Technik weitere Elemente im Kraftfluss, nämlich die Verbindungsglieder 15, zwischengeschaltet, welche konstruktiv durch die beiden Langlöcher 14 bewirkt werden.

Figur 7 zeigt eine erfindungsgemäße Schlauchklemme einer weiteren, zweiten bevorzugten Ausführungsform. Hierbei sind die Aussparungen different geometrisch ausgeformt und weisen neben einem Langloch quer zur Klemmenlängsrichtung L auch noch eine an das Langloch sich anschließende Aussparung in Klemmenlängsrichtung L auf. Durch die Formgebung der Aussparung lässt sich eine Elastizität der zweiten Klemmkante 6b gegenüber dem ersten Klemmabschnitt 4b weiter ändern bzw. definieren.

An dieser Stelle sei angemerkt, dass natürlich statt des Scharniers 5 mit einhergehender Schwenkbewegung um die Schwenkachse S natürlich auch eine translatorische Bewegung der beiden Klemmabschnitte 4a, 4b aufeinander zu realisiert werden kann. Der technische Effekt der Verbindungsglieder 15 und der elastischen Kopplung ist hierbei wieder der gleiche wie oben beschrieben.

### Bezugszeichen

- 1: Schlauchklemme
- 2: Pumpe
- 2.1: Gehäuse
- 2.2: Gegenanlagefläche
- 3: Schlauch
- 4a: erster Klemmabschnitt
- 4b: zweiter Klemmabschnitt
- 5: Scharnier
- 6a: erste Klemmbacke
- 6b: zweite Klemmbacke
- 7: Schlauchaufnahmebereich
- 8: Steg
- 9: Ausnehmung
- 10: Verschlusssystem
- 11: Schnapphaken
- 12: Schnappöse
- 13: Druckknopfloch
- 14: Aussparung
- 15: Verbindungsglied
- 16: Anlagefläche

- S: Schwenkachse
- L: Klemmenlängsrichtung
- A: Schlauchlängsachse
- D: Klemmdruckrichtung

## Patentansprüche

1. Medizinische Schlauchklemme (1) zum Klemmen eines flexiblen medizinischen Schlauchs (3), mit:
einem Schlauchaufnahmebereich (7), der dafür angepasst ist, den medizinischen Schlauch (3) aufzunehmen,
einem ersten Klemmabschnitt (4a) mit einer ersten Klemmbacke (6a), der gegenüber einem zweiten Klemmabschnitt (4b) mit einer zweiten Klemmbacke (6b) bewegbar ist, wobei die erste Klemmbacke (6a) und zweite Klemmbacke (6b) jeweils in Form einer wandförmigen Klemmkante ausgebildet ist, und
einem Verschlusssystem (10) zum sicheren Positionieren der beiden Klemmabschnitte (4a, 4b) in mehr als einer Rast-Stellung zueinander, so dass bei unverrastetem Verschlusssystem (10) der medizinische Schlauch (3) in die medizinische Schlauchklemme (1) einlegbar und entnehmbar ist, in einer Offen-Raststellung der eingelegte medizinische Schlauch (3) nicht entnehmbar ist und nicht geklemmt wird und in einer Klemm-Raststellung der medizinische Schlauch (3) zwischen der ersten Klemmbacke (6a) und der zweiten Klemmbacke (6b) geklemmt wird,
**dadurch gekennzeichnet, dass**
die erste Klemmbacke (6a) über ein Verbindungsglied (15) elastisch beweglich zu dem ersten Klemmabschnitt (4a) und/oder die zweite Klemmbacke (6b) über ein Verbindungsglied (15) elastisch beweglich zu dem zweiten Klemmabschnitt (4b) ausgebildet ist.

2. Medizinische Schlauchklemme (1) nach Anspruch 1, **dadurch gekennzeichnet, dass** in der Klemm-Raststellung der erste Klemmabschnitt (4a) durch das Verschlusssystem (10) starr gegenüber dem zweiten Klemmabschnitt (4b) positioniert ist und die erste Klemmbacke (6a) und/oder die zweite Klemmbacke (6b) im Wesentlichen nur in Klemmdruckrichtung (D) elastisch beweglich ist.

3. Medizinische Schlauchklemme (1) nach Anspruch 1 oder 2, **dadurch gekennzeichnet, dass** die medizinische Schlauchklemme (1) ein Scharnier (5), insbesondere ein Filmscharnier, aufweist, durch das der erste Klemmabschnitt (4a) relativ zu dem zweiten Klemmabschnitt (4b) um eine Schwenkachse (S) schwenkbar ist, so dass die medizinische Schlauchklemme (1) sich maulartig öffnen und schließen lässt.

4. Medizinische Schlauchklemme (1) nach Anspruch 1 oder 2, **dadurch gekennzeichnet, dass** die medizinische Schlauchklemme (1) einen Mechanismus aufweist, mit dem der zweite Klemmabschnitt (4b) nur einen translatorischen Freiheitsgrad in Richtung des ersten Klemmabschnitts (4a) aufweist und bewegbar ist.

5. Medizinische Schlauchklemme (1) nach einem der vorangegangenen Ansprüche, **dadurch gekennzeichnet, dass** das Verschlusssystem (10) in Form eines Schnappverschlusssystem/Schnappeinrichtung ausgebildet ist.

6. Medizinische Schlauchklemme (1) nach einem der vorangegangenen Ansprüche, **dadurch gekennzeichnet, dass** der erste Klemmabschnitt (4a) und/oder der zweite Klemmabschnitt (4b) einen plattenförmigen Grundkörper aufweist, der im vorzugsweise direkt angrenzenden Bereich an die jeweilige Klemmbacke (6a, 6b) zumindest eine Aussparung (14), insbesondere in Form eines Langlochs, aufweist, wodurch ein Verbindungsglied (15) im Grundkörper ausgebildet wird.

7. Medizinische Schlauchklemme (1) nach Anspruch 6, **dadurch gekennzeichnet, dass** die Klemmbacken (6a, 6b) als Klemmkanten ausgebildet sind und der erste und/oder der zweite Klemmabschnitt (4a, 4b) zwei Langlöcher als Aussparungen (14) aufweist, deren Längsrichtung quer zu einer Klemmenlängsrichtung (L) liegt und die Langlöcher jeweils direkt an die jeweilige Klemmkante (6a, 6b) angrenzen.

8. Medizinische Schlauchklemme (1) nach einem der vorangegangenen Ansprüche, **dadurch gekennzeichnet, dass** der erste Klemmabschnitt (4a), das Verbindungsglied (15) und die erste Klemmbacke (6a) stoffeinstückig ausgebildet sind und/oder der zweite Klemmabschnitt (4b), das Verbindungsglied (15) und die zweite Klemmbacke (6b) stoffeinstückig ausgebildet sind.

9. Medizinische Schlauchklemme (1) nach einem der vorangegangenen Ansprüche, **dadurch gekennzeichnet, dass** das Verbindungsglied ein elastisches Material, eine Feder oder ein Vorspannmechanismus ist.

10. Volumetrische Pumpe (2), insbesondere eine Infusionspumpe, zum Fördern eines Mediums durch einen flexiblen medizinischen Schlauch (3), mit einem Gehäuse (2.1), **dadurch gekennzeichnet, dass** an dem Gehäuse (2.1) zum Klemmen des medizinischen Schlauchs (3) eine medizinische Schlauchklemme (1) nach einem der Ansprüche 1 bis 9, vorzugsweise lösbar, befestigt ist.

## Claims

1. A medical tube clamp (1) for clamping a flexible medical tube (3), comprising:
a tube receiving area (7) which is adapted to receive the medical tube (3),
a first clamping portion (4a) including a first clamping jaw (6a) which is movable relative to a second clamping portion (4b) including a second clamping jaw (6b), wherein the first clamping jaw (6a) and the second clamping jaw (6b) are respectively formed as a wall-shaped clamping edge, and
a closure system (10) for safely positioning the two clamping portions (4a, 4b) in more than one snap-fit position relative to each other so that, when the closure system (10) is unlocked, the medical tube (3) can be inserted in and removed from the tube clamp (1), in an open snap-fit position the inserted medical tube (3) cannot be removed and is not clamped, and in a clamping snap-fit position the medical tube (3) is clamped between the first clamping jaw (6a) and the second clamping jaw (6b),
**characterized in that**
the first clamping jaw (6a) is configured to be elastically movable relative to the first clamping portion (4a) via a connecting member (15) and/or the second clamping jaw (6b) is configured to be elastically movable relative to the second clamping portion (4b) via a connecting member (15).

2. The medical tube clamp (1) according to claim 1, **characterized in that** in the clamping snap-fit position, the first clamping portion (4a) is positioned rigidly vis-à-vis the second clamping portion (4b) by the closure system (10) and the first clamping jaw (6a) and/or the second clamping jaw (6b) is/are elastically movable substantially in the clamping pressure direction (D) only.

3. The medical tube clamp (1) according to claim 1 or 2, **characterized in that** the tube clamp (1) includes a hinge (5), especially a film hinge, by which the first clamping portion (4a) is pivoting relative to the second clamping portion (4b) about a pivot axis (S) so that the medical tube clamp (1) can be opened and closed in a jaw-type manner.

4. The medical tube clamp (1) according to claim 1 or 2, **characterized in that** the tube clamp (1) has a mechanism by which the second clamping portion (4b) has only a translational degree of freedom and is movable in the direction of the first clamping portion (4a).

5. The medical tube clamp according to one of the preceding claims, **characterized in that** the closure system (10) is in the form of a snap-fit closure system/snap-fit.

6. The medical tube clamp (1) according to one of the preceding claims, **characterized in that** the first clamping potion (4a) and/or the second clamping portion (4b) have/has a plate-shaped base which in the area preferably directly abutting on the respective clamping jaw (6a, 6b) includes at least one recess (14), especially in the form of a slotted hole, thus causing a connecting member (15) to be formed in the base.

7. The medical tube clamp (1) according to claim 6, **characterized in that** the clamping jaws (6a, 6b) are in the form of clamping edges and the first and/or second clamping portion(s) (4a; 4b) have/has two slotted holes (14) the longitudinal direction of which is located transversely to a longitudinal clamp direction (L) and the slotted holes are directly abutting on the respective clamping edge (6a, 6b).

8. The medical tube clamp (1) according to one of the preceding claims, **characterized in that** the first clamping portion (4a), the connecting member (15) and the first clamping jaw (6a) are made from one material and/or the second clamping portion (4b), the connecting member (15) and the second clamping jaw (6b) are made from one material.

9. The medical tube clamp according to one of the preceding claims, **characterized in that** the connecting member is an elastic material, a spring or a preloading mechanism.

10. A volumetric pump (2), especially an infusion pump, for delivering a medium through a flexible medical tube (3), comprising a housing (2.1.), **characterized in that** a tube clamp (1) according to one of the claims 1 to 9 is, preferably detachably, fastened on the housing (2.1) for clamping the medical tube (3).

## Revendications

1. Collier de serrage médical (1) pour le serrage d'un tuyau médical souple (3), avec :
une zone d'accueil de tuyau (7) qui est adaptée pour accueillir le tuyau médical (3),
une première section de serrage (4a) avec une première mâchoire de serrage (6a) qui est mobile par rapport à une seconde section de serrage (4b) avec une seconde mâchoire de serrage (6b), dans lequel la première mâchoire de serrage (6a) et la seconde mâchoire de serrage (6b) sont respectivement réalisées en forme d'arête de serrage en forme de paroi, et
un système de verrouillage (10) pour le positionnement sécurisé des deux sections de serrage (4a, 4b) dans plus qu'une position d'enclenchement l'une par rapport à l'autre, de sorte que dans un système de verrouillage non enclenché (10) le tuyau médical (3) est insérable dans le collier de serrage médical (1) et en est retirable, dans une position d'enclenchement ouverte le tuyau médical (3) inséré n'est pas retirable et n'est pas serré et dans une position d'enclenchement par serrage le tuyau médical (3) entre la première mâchoire de serrage (6a) et la seconde mâchoire de serrage (6b) le tuyau médical (3) est serré,
**caractérisé en ce que**
la première mâchoire de serrage (6a) est réalisée élastiquement de manière mobile par rapport à la première section de serrage (4a) par le biais d'un organe de liaison (15) et/ou la seconde mâchoire de serrage (6b) est réalisée élastiquement de manière mobile par rapport à la seconde section de serrage (4b) par le biais d'un organe de liaison (15).

2. Collier de serrage médical (1) selon la revendication 1, **caractérisé en ce que** dans la position d'enclenchement par serrage la première section de serrage (4a) est positionnée de manière rigide par rapport à la seconde section de serrage (4b) par l'intermédiaire du système de verrouillage (10) et la première mâchoire de serrage (6a) et/ou la seconde mâchoire de serrage (6b) sont mobiles élastiquement pour l'essentiel uniquement dans la direction de poussée de serrage (D).

3. Collier de serrage médical (1) selon la revendication 1 ou 2, **caractérisé en ce que** le collier de serrage médical (1) présente une charnière (5), en particulier une charnière-film, à travers laquelle la première section de serrage (4a) peut pivoter par rapport à la seconde section de serrage (4b) autour d'un axe de pivotement (S), de sorte que le collier de serrage médical (1) s'ouvre et se ferme comme une bouche.

4. Collier de serrage médical (1) selon la revendication 1 ou 2, **caractérisé en ce que** le collier de serrage médical (1) présente un mécanisme avec lequel la seconde section de serrage (4b) ne présente et n'est mobile que selon un degré de liberté de translation dans la direction de la première section de serrage (4a).

5. Collier de serrage médical (1) selon l'une des revendications précédentes, **caractérisé en e que** le système de verrouillage (10) est réalisé en forme de système de verrouillage à déclic/dispositif à déclic.

6. Collier de serrage médical (1) selon l'une des revendications précédentes, **caractérisé en ce que** la première section de serrage (4a) et/ou la seconde section de serrage (4b) présentent un corps de base en forme de plaque qui présente dans la zone de préférence directement adjacente à la mâchoire de serrage (6a, 6b) respective au moins une cavité (14), en particulier en forme de trou longitudinal, moyennant quoi un organe de liaison (15) est réalisé dans le corps de base.

7. Collier de serrage médical (1) selon la revendication 6, **caractérisé en ce que** les mâchoires de serrage (6a, 6b) sont réalisées en tant qu'arêtes de serrage et la première et/ou la seconde section de serrage (4a, 4b) présentent deux trous longitudinaux en tant que cavités (14), dont la direction longitudinale est transversale à une direction longitudinale de serrage (L) et les trous longitudinaux sont respectivement directement adjacents à l'arête de serrage (6a, 6b) respective.

8. Collier de serrage médical (1) selon l'une des revendications précédentes, **caractérisé en ce que** la première section de serrage (4a), l'organe de liaison (15) et la première mâchoire de serrage (6a) sont réalisés d'un seul tenant et/ou la seconde section de serrage (4b), l'organe de liaison (15) et la seconde mâchoire de serrage (6b) sont réalisés d'un seul tenant.

9. Collier de serrage médical (1) selon l'une des revendications précédentes, **caractérisé en ce que** l'organe de liaison est un matériau élastique, un ressort ou un mécanisme de précontrainte.

10. Pompe volumétrique (2), en particulier pompe de perfusion, pour le refoulement d'un milieu à travers un tuyau médical souple (3), avec un boîtier (2.1), **caractérisée en ce qu'**au niveau du boîtier (2.1) destiné au serrage du tuyau médical (3) un collier de serrage médical (1) selon l'une des revendications 1 à 9, est fixé, de préférence de manière amovible.
